(19) Europäisches Patentamt
European Patent Office
Office européen des brevets

(11) **EP 2 402 387 B1**

(12) **FASCICULE DE BREVET EUROPEEN**

(45) Date de publication et mention de la délivrance du brevet:
**16.04.2014   Bulletin 2014/16**

(21) Numéro de dépôt: **11173204.6**

(22) Date de dépôt: **11.12.2008**

(51) Int Cl.:
*C08K 3/22* $^{(2006.01)}$          *A61K 8/02* $^{(2006.01)}$
*A61K 41/00* $^{(2006.01)}$        *A61F 7/08* $^{(2006.01)}$
*D01F 6/60* $^{(2006.01)}$         *C08K 3/18* $^{(2006.01)}$
*C08K 3/30* $^{(2006.01)}$         *C08K 3/32* $^{(2006.01)}$
*C08K 3/34* $^{(2006.01)}$         *A61K 8/23* $^{(2006.01)}$
*A61K 8/26* $^{(2006.01)}$         *A61K 8/29* $^{(2006.01)}$
*A61K 8/88* $^{(2006.01)}$         *A61Q 19/06* $^{(2006.01)}$
*D01F 1/10* $^{(2006.01)}$         *C08J 3/22* $^{(2006.01)}$
*A61F 13/06* $^{(2006.01)}$        *A61K 31/785* $^{(2006.01)}$
*A61K 9/00* $^{(2006.01)}$

(54) **Utilisation d'un article à base d'une composition polymérique**

Verwendung eines Artikels basierend auf einer Polymerzusammensetzung

Use of an article based on a polymeric composition

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **14.12.2007   FR 0708724**
**30.07.2008   FR 0804334**

(43) Date de publication de la demande:
**04.01.2012   Bulletin 2012/01**

(62) Numéro(s) de document de la (des) demande(s) initiale(s) en application de l'article 76 CBE:
**08862080.2 / 2 220 145**

(73) Titulaire: **Rhodia Poliamida E Especialidades Ltda Sao Paulo - SP (BR)**

(72) Inventeurs:
• **Cordeiro Bastos, Tarcis**
  **CEP 04014-011 São Paulo (SP) (BR)**
• **Canova, Thomas**
  **CEP 09040-270 São Paulo (SP) (BR)**
• **Bizaroli de Mendonça, Dany**
  **SP São Paulo (SP) (BR)**

(74) Mandataire: **Casalonga, Axel et al**
**Casalonga & Partners**
**Bayerstrasse 73**
**80335 München (DE)**

(56) Documents cités:
EP-A- 1 291 405          WO-A-2007/055432
WO-A1-02/087700        US-A- 4 999 243
US-A1- 2007 141 095      US-B1- 6 316 102

• 'Causes of Orange peel skin', [en ligne] 07 Mai 2013, Extrait de l'Internet: <URL:http: //www.rightdiagnosis.com/symptoms /orange_ peel_skin/causes.htm> [extrait le 2013-07-23]

**Description**

La présente invention se rapporte à une composition polymérique qui comprend l'utilisation d'additifs ayant des propriétés d'émission et/ou absorption de radiation dans la région de l'infrarouge long, ainsi qu'à des articles fabriqués à partir de cette composition.

Plus spécifiquement, la présente invention se rapporte à une composition polymérique qui comprend des additifs ayant des propriétés d'émission de radiation dans la région infrarouge, dans une plage de longueur d'onde située entre $2\mu m$ et $20\mu m$, ainsi qu'à des articles fabriqués à partir de cette composition. La présente invention se rapporte également à des procédés de fabrication de fils et compositions de polyamide contenant ces additifs, ainsi qu'à des articles tels que des articles textiles comme des tissus ou des tricots fabriqués à partir de ces fils, et l'utilisation de ces articles.

L'interaction entre la radiation dans la région infrarouge, de longueur d'onde entre $2\mu m$ et $20\mu m$, et les tissus biologiques attire l'attention des scientifiques depuis deux décennies. D'après les études publiées, la radiation infrarouge dans cette plage entraîne la biostimulation telle que l'augmentation de la microcirculation du sang, la diminution de spasmes musculaires, l'augmentation du métabolisme cellulaire, entre autres. Selon l'un des mécanismes proposés, les cellules des tissus biologiques sont stimulées par un processus de résonance avec la radiation, entraînant une augmentation de la circulation sanguine et une diminution de la concentration d'acide lactique dans les muscles squelettiques humains (Niwa et al 1993: Niwa, Y.; Iizawa O.; Ishimoto K.; Jiang. X.; Kanoh, T.; Electromagnetic Wave Emitting products and "Kikoh" Potentiate Human Leukocyte Functions; International Journal of Biometeorology n° 37, p.133-138, 1993; Perez et Martinez 1995: Pérez, A. C. N., Martinez, A. J. A., Fibra de Photon Plantino. Saint Jean de Compostelle, 1995, p.7-71). D'autres effets tels que l'augmentation du flux sanguin périphérique, l'augmentation de la température du corps ont également été décrits dans la littérature.

Au cours des dernières années, des brevets ont été publiés, revendiquant l'utilisation de matériaux émetteurs de radiation infrarouge dans la plage considérée ci-dessus, pour une application textile. En général, l'application est dirigée vers les effets d'absorption thermique (US5053275), antimicrobiens (US6316102) et concerne l'utilisation de particules de titane métallique (US7201945) ou d'une composition de charges minérales d'oxydes, carbures, sulfates et silicates. Les matériaux cités dans les brevets sont appliqués au moyen d'une solution aqueuse (dans le cas du titane métallique) ou alors mélangés et traités avec un type de résine polymérique et déposés en enduction (« coatings ») (EP1792724) sur des surfaces textiles. Ces applications superficielles ne présentent pas de bonne résistance à l'usage et au lavage, ni de toucher agréable au contact de la peau, notamment sous la forme d'enduction (« coatings ») de résines. Certains brevets cherchent à résoudre ce problème en incorporant les matériaux dans le substrat polymérique et en produisant des filaments au moyen de procédés d'extrusion, étirage et texturation (US4999243, US5880044, WO2007/055432). Cependant, l'utilisation de taux élevés d'oxydes et de carbures de dureté élevée n'est pas adéquate pour la production de fils à partir de composants thermoplastiques, car ils occasionnent une rapide détérioration des organes des machines. La solution trouvée présente également des inconvénients: la coloration de certains carbures et la faible efficacité du traitement (fréquentes ruptures de filaments) compromettent les propriétés mécaniques du fil.

Une alternative proposée par le brevet EP1094136 concerne l'utilisation d'une composition de particules conductrices blanches, d'oxydes blancs émetteurs d'infrarouge et de résines thermoplastiques pour la production de filaments ayant des taux d'oxydes plus bas. Cependant, la composition présente des oxydes de dureté très élevée (au-dessus de 8,0Mohs et l'utilisation de titanate de potassium, qui se présente en général sous forme d'une poudre fibreuse, pouvant être classée comme fibre respirable, rend la manipulation difficile et désavantageuse du point de vue de l'hygiène et de la santé.

Par ailleurs, on est toujours à la recherche de produits cosmétiques qui stimulent la peau, en particulier pour des personnes ayant de la « cellulite » (lipodystrophie gynoïde). En effet la « cellulite » est généralement liée à la présence de corps gras dans les cellules grasses présentes sous la peau, qui entraîne une distorsion des tissus sous la peau, .et provoque le fameux effet « peau d'orange ». On cherche donc toujours des solutions pour diminuer la cellulite. Ceci afin d'améliorer le confort et le bien-être de la personne.

A cet effet il a été trouvé dans le cadre de la présente invention que des additifs ayant des propriétés d'émission et/ou d'absorption dans la région de l'infrarouge interagissent également avec un autre tissu biologique qui est la peau. En effet ces additifs permettent notamment de diminuer voire de supprimer la cellulite, ce qui est particulièrement intéressant.

La présente invention se rapporte à la production d'une composition polymérique dans laquelle les caractéristiques des additifs absorbeurs et/ou émetteurs d'infrarouge (dans la plage de longueur d'onde située entre $2\mu m$ et $20\mu m$) et les taux utilisés résolvent les problèmes posés plus haut concernant la difficulté de traitement des charges minérales et des fils, permettant la production de fils et d'articles textiles offrant confort, bien-être, amélioration de la microcirculation, meilleure homogénéité thermique et diminution de la fatigue musculaire.

Le principal objectif de la présente invention est d'obtenir des fils, fibres, filaments et articles ayant des propriétés de stimulation de la microcirculation sanguine pour offrir une meilleure homogénéité thermique et une diminution de la fatigue musculaire, ainsi qu'une meilleure élasticité de la peau, grâce à l'introduction, dans une matrice polymérique, d'additifs ayant une propriété d'émission et/ou absorption d'infrarouge, de manipulation facile et aisée d'un point de vue industriel. Ces articles permettent notamment de diminuer la cellulite.

La présente invention se rapporte à l'emploi de charges minérales introduits dans des polymères pour conférer des propriétés d'émission d'infrarouge capables d'offrir une meilleure homogénéité thermique et une meilleure élasticité de la peau, une biostimulation pour une diminution de la fatigue musculaire, afin d'apporter confort et bien-être à la personne ; ainsi qu'au procédé pour l'obtention en particulier des fibres, fils et articles obtenus à partir de ces compositions polymériques. Les polymères utilisés sont ceux filés à l'état fondu tels que le polyester, le polyamide, les polyoléfines (et leurs copolymères), entre autres, ou à travers des solutions, tels que les polymères polyacryliques, les polyacrylates et leurs copolymères, et les dérivés de cellulose tels que l'acétate de cellulose, le propionate de cellulose, la viscose, etc. Les additifs peuvent être introduits dans le polymère selon une méthode quelconque connue de l'homme du métier. Préférentiellement, l'introduction est réalisée dans la phase de synthèse du polymère, ou bien dans la phase de filage au moyen d'un mélange direct des charges minérales dans le polymère fondu ou en solution, ou encore à travers un mélange maître (« masterbatch »), l'utilisation d'une combinaison de deux modes d'introduction pouvant être appropriée.

La composition selon l'invention comprend une combinaison de charges minérales qui présentent une capacité d'émission et/ou absorption de radiations infrarouges dans la plage de longueur d'onde située entre $2\mu$m et $20\mu$m, et d'un polymère.

La composition selon l'invention présente un nombre de pics d'absorption de radiations infrarouges supérieur à 10 dans les dix plages de fréquence suivantes : 3,00 +/- 0,30$\mu$m, 6,20 +/- 0,50$\mu$m, 8,00 +/- 0,25$\mu$m, 8,50 +/- 0,25$\mu$m, 9,00 +/- 0,25$\mu$m, 9,50 +/- 0,25$\mu$m, 10,00 +/- 0,25$\mu$m, 10,50 +/- 0,25$\mu$m, 11,00 +/- 0,25$\mu$m, 14,60 +/- 2,10$\mu$m, au moins 1 pic étant présent dans au moins 7 de ces dix plages de fréquence.

Le spectre d'absorption de radiations infrarouges de la composition peut être déterminé par toute méthode connue de l'homme du métier. Une méthode possible est l'utilisation d'un appareil Bruker Equinox 55, avec une résolution de 4 cm$^{-1}$. Dans ce cas le spectre obtenu est sous forme ATR (« Atenuated Total Reflectance »), en utilisant un cristal ZnSe.

Selon l'invention, les charges minérales sont d'au moins un type choisi parmi les oxydes, sulfates, carbonates, phosphates et silicates, présentant une taille moyenne de particule inférieure à $2\mu$m.

Selon la présente invention, on fournit une composition de polymère qui inclut des additifs émetteurs d'infrarouge dans la plage de longueur d'onde située entre $2\mu$m et $20\mu$m. Le polymère peut être choisi dans le groupe comprenant les polyesters, les polyoléfines, les polymères à base de cellulose-ester tels que l'acétate de cellulose, le propionate de cellulose, le rayon, la viscose et les polymères de la même famille, les polymères et copolymères acryliques, les polyamides, le polyhexaméthylène adipamide (PA66) ou le polycaproamide (PA6), ou leurs copolymères en toutes proportions, ou encore des mélanges entre n'importe quels polymères cités précédemment. Selon une forme préférentielle de réalisation, le polymère thermoplastique qui compose la matrice thermoplastique de la composition polymérique est à base de polyamide, choisi entre le polyamide 6, le polyamide 66 et les copolymères de polyamide 6/polyamide 66 en toutes proportions.

Des additifs ont été développés, et qui peuvent être utilisés dans la production par exemple de fils, fibres et filaments ayant des propriétés biostimulantes qui offrent une amélioration de la microcirculation sanguine, une meilleure homogénéité thermique, une meilleure élasticité de la peau et une diminution de la fatigue musculaire, résultant en un plus grand confort et bien-être pour les utilisateurs des articles les contenant, en particulier les utilisateurs ayant de la cellulite. Ces articles permettent de diminuer la cellulite.

Plus précisément, la présente invention se rapporte en premier lieu à l'utilisation d'une association d'additifs dans des compositions polymériques pour obtenir l'effet décrit ci-dessus, caractérisé en ce que l'association comprend au moins une charge minérale choisie parmi le groupe des oxydes (dioxyde de titane, dioxyde de silicium, oxyde de magnésium), le groupe des sulfates (sulfate de baryum, sulfate de calcium, sulfate de strontium), le groupe des carbonates (carbonate de calcium ou de sodium), le groupe des silicates (actinolite, tourmaline, serpentine, kaolin et autres aluminosilicates) et le groupe des phosphates (phosphates de zirconium, apatite, ainsi que d'autres possibles, ou encore leurs mélanges).

Les charges minérales utilisées en association comme absorbeurs et/ou émetteurs d'infrarouge dans la plage de longueur d'onde située entre $2\mu$m et $20\mu$m se présentent sous forme de particules de taille inférieure à $2\mu$m, préférentiellement inférieure à $1\mu$m et avantageusement inférieure à $0,5\mu$m. Les particules peuvent être avantageusement enveloppées ou recouvertes pour les rendre inertes aux composants auxquels elles seront incorporées ou encore pour procurer une meilleure compatibilité avec le substrat polymérique, sans que cela n'intervienne dans leur caractéristique d'absorbeurs et/ou émetteurs d'infrarouge dans la plage considérée.

Les associations de deux charges minérales, ou de trois charges minérales, sont préférées, et en particulier les associations ternaires peuvent être choisies parmi celles qui comprennent le dioxyde de titane, le sulfate de baryum, le dioxyde de silicium et une charge du groupe des silicates.

Encore plus particulièrement, l'association comprend trois charges minérales en mélange de proportions quelconques, telles que celles choisies dans le groupe comprenant : dioxyde de titane/ dioxyde de silicium/ tourmaline ; dioxyde de titane/ dioxyde de silicium/ sulfate de baryum; et dioxyde de titane/ sulfate de baryum/ tourmaline. Préférentiellement on emploie le dioxyde de titane/ sulfate de baryum/ tourmaline.

Selon la présente invention, l'association de charges minérales décrite ci-dessus est utilisée comme additif émetteur d'infrarouge dans la plage de $2\mu m$ à $20\mu m$ dans des compositions polymériques pour la production de fils, fibres, filaments et articles textiles.

L'additif est présent selon l'invention en quantité inférieure à 6,0% d'additif par rapport à la masse totale de la composition de polymère, de préférence inférieure à 4,5% en poids. De même, selon un autre mode particulier de réalisation de l'invention, la proportion en poids de l'association de charges minérales par rapport au poids total de la composition polymérique est supérieure à 1,0%, de préférence supérieure ou égale à 1,5% et plus préférentiellement encore supérieure ou égale a 2,5%.

La composition de polymère peut encore contenir un agent antimicrobien ou bactériostatique, ignifugeant, stabilisant face aux rayons UV, ainsi que d'autres agents connus de l'homme du métier.

Selon la présente invention, il est possible d'utiliser une association d'additifs telle que celle décrite ci-dessus en proportions quelconques. À titre d'exemple, et de manière non limitative, les charges minérales des associations ternaires seront réalisées dans l'utilisation de la présente invention en proportions variant avantageusement de 80:10:10 à 10:30:60, plus spécifiquement en proportions de 50:25:25.

Un autre objet de la présente invention est le procédé de préparation des compositions polymériques avec une association de charges minérales absorbeuses/émettrices d'infrarouge distant tel que défini précédemment. Les charges ou additifs peuvent êtres introduits dans la composition polymérique selon une méthode quelconque connue de l'homme du métier. Préférentiellement, l'introduction est réalisée au cours de la phase de synthèse du polymère, ou par mélange directement au polymère au cours de la phase de filage des filaments, ou encore au moyen d'un concentré de particules sous forme de « masterbatch », postérieurement dilué en concentrations prédéterminées dans la masse polymérique au cours de la phase de filage.

Les charges minérales peuvent être additionnés séparément selon l'une ou plusieurs méthodes d'introduction décrites ci-dessus.

Le mélange maître est préparé avec des quantités de charge minérale comprises avantageusement entre 10% et 65% en poids par rapport à sa masse totale, de préférence entre 15% et 35%, encore plus préférentiellement entre 15% et 25%.

La présente invention se rapporte également aux articles et en particulier aux fils, fibres et filaments obtenus à partir des compositions décrites ci-dessus, dans lesquelles l'association des charges minérales de la présente invention a été utilisée.

Dans le cas de fils, fibres et filaments obtenus par filage à l'état fondu, la composition thermoplastique additivée est obtenue avec l'introduction des charges minérales dans le polymère fondu au moyen d'un dispositif de mélange, par exemple en amont d'un dispositif de filage. Par le filage de la composition thermoplastique additivée on peut obtenir des fils multifilamentaires continus, des monofilaments, des fibres courtes et longues, ou leurs mélanges. Les fils, fibres et filaments obtenus à partir des compositions polymériques présentées dans la présente invention peuvent être soumis à tous les traitements textiles connus de l'homme du métier, tels qu'extrusion, étirage, texturation, teinture, finition etc.

La présente invention se rapporte également aux articles obtenus à partir des fils, fibres et filaments décrits ci-dessus. Les articles peuvent être obtenus à partir d'un seul type de fil, fibre ou filament, ou à partir d'un mélange de fils, fibres ou filaments de types différents.

Par articles, on entend notamment les tissus, les tricots et les non-tissés. L'article peut être composé d'au moins un type de fil, filament ou fibre obtenu à partir de compositions polymériques décrites dans la présente invention.

L'article peut également être un film ou une poudre obtenus à partir de la composition décrite ci-dessus. Le film ou la poudre peuvent être obtenus selon toute méthode connue de l'homme du métier.

La présente invention se rapporte à l'utilisation d'un article, en particulier textile, tel que décrit ci-dessus, à base d'une composition telle que décrite ci-dessus, pour stimuler des tissus biologiques, en particulier des tissus biologiques de sportifs. Avantageusement le tissu biologique est la peau, en particulier la peau de personnes ayant de la cellulite. Selon un mode de réalisation particulier de l'invention, l'invention concerne une telle utilisation pour diminuer la cellulite des personnes.

Tout ce qui a été décrit ci-dessus concernant la composition polymérique de l'invention et les articles de l'invention s'applique ici pour l'utilisation de l'invention.

Les exemples suivants présentés à titre indicatif feront bien ressortir les avantages de la présente invention.

## EXEMPLES

Les échantillons des exemples 1 à 6 ci-dessous ont été préparés avec un polyamide 66 de viscosité relative (VR) 43, mesurée dans une solution d'acide formique à 90% dans l'eau. L'incorporation de charges minérales émettrices d'infrarouge dans le polyamide 66 a été réalisée à travers le mélange des charges minérales sous forme de poudre et du polymère trituré, dans une proportion de 20% en poids de charge minérale pour l'obtention d'un mélange maître. Le mélange a été extrudé, refroidi et granulé. Le « masterbatch » ainsi obtenu a été introduit dans le polyamide 66 au cours de la phase de filage. La composition polymérique fondue a été filée à une température entre 280°C et 300°C (mesurée dans la filière), refroidie à l'air (20°C, humidité relative de 65%) et enroulée à une vitesse de 4200m/min pour obtenir un fil continu multifilamentaire. Le fil multifilamentaire formé de 68 filaments de section circulaire a été postérieurement texturé. Le titre du filament dans le produit fini est de 1,2dtex. Le fil ainsi obtenu a été utilisé dans la production de tricots pour la confection de bermudas et tee-shirts, par utilisation d'une tricoteuse circulaire. Les tee-shirts ainsi obtenus présentent une densité de surface de 175 g/m$^2$, et les bermudas une densité de surface de 305 g/m$^2$, et contiennent 12% d'élasthanne. Ces articles ont ensuite été utilisés pour évaluer la performance des compositions.

**Exemple 1**

Un échantillon de fil de polyamide 66 contenant 1,5% de Ti02 et 0,5% de BaS04 a été préparé selon la description précédente. On a comparé la variation thermique du corps (tronc et membres inférieurs) de deux groupes de 15 athlètes soumis à l'application d'un protocole d'activité physique (test ergométrique en tapis de course selon le protocole de Bruce). Le test a été effectué au cours de trois jours:

- le jour 1, les athlètes ont été soumis au protocole de Bruce vêtus d'un ensemble de tee-shirt en coton et bermuda en polyester, appelé groupe contrôle, pour la définition du temps maximal (t) (défini comme la durée d'activité physique jusqu'à l'atteinte d'une certaine limite de fréquence cardiaque ou de pression artérielle -pré-définies selon l'âge de la personne-, ou jusqu'à une demande d'arrêt de l'athlète par fatigue)
- le jour 2, les athlètes n'ont pratiqué aucune activité physique;
- le jour 3, les mêmes athlètes ont été soumis au protocole de Bruce jusqu'à atteindre le temps (t), vêtus de l'ensemble de tee-shirt et bermuda, appelé groupe échantillon;
- échantillons évalués: coton et polyamide 66 contenant 1,5% de Ti02 et 0,5% de BaS04.

La température du corps a été mesurée au moyen de la technique de thermographie (équipement Raytec Fluke TiSO Thermal Image) avant et après l'application du protocole, et l'indice de variation thermique $\Delta T/\Delta T1$ a été évalué.

L'indice de variation thermique a été obtenu par la comparaison des températures moyennes avant (température initiale) et après (température finale) la réalisation du protocole d'activités physiques:

$$\Delta T = \Delta T2 - \Delta T1$$

où:

$\Delta T1 = T_{fc} - T_{lc}$ (soustraction entre les températures moyennes finales $T_{fc}$ et initiales $T_{lc}$ du groupe contrôle), et
$\Delta T2 = T_{fe} - T_{le}$ (soustraction entre les températures moyennes finales $T_{fe}$ et initiales $T_{le}$ du groupe échantillon).

Le tableau 1 ci-dessous résume les indices de variation thermique $\Delta T/\Delta T1$ obtenus dans les régions du tronc et latérale de la jambe pour le groupe A (athlètes vêtus de tee-shirt et bermuda en coton) et B (athlètes vêtus de tee-shirt et bermuda en PA66 contenant 1,5% de Ti02 et 0,5% de BaS04).

<div align="center">Tableau 1</div>

| Groupe | Échantillon | Tronc $\Delta T/\Delta T1$ (%) | Latérale jambe $\Delta T/\Delta T1$ (%) |
|--------|-------------|-------------------------------|------------------------------------------|
| A | Coton | 38 | 48 |
| B | Polyamide 66 + TiO2 + BaSO4 | 48 | 62 |

Les indices obtenus montrent une élévation de la température dans le groupe B comparée au groupe A, indiquant une augmentation de la circulation sanguine lorsque sont utilisés les articles fabriqués en polyamide additivé.

## Exemple 2

Un échantillon de fil de polyamide 66 contenant 1,5% de Ti02 et 0,5% de BaS04 a été préparé selon la description précédente et comparé à un échantillon de fil polyamide contenant 1,5% de Ti02. On a comparé la variation thermique du corps (tronc et membres inférieurs) de deux groupes de 15 athlètes soumis à l'application d'un protocole d'activité physique (test ergométrique en tapis de course selon le protocole de Bruce). Le test et la mesure de température ont été effectués tels que décrits dans l'exemple 1.

L'indice d'hétérogénéité thermique (h) a été obtenu par le calcul de la variation de l'écart des mesures de température de la région considérée à la suite de l'application du protocole d'activité physique:

$$h = (d2 - d1) / d1$$

où,

d1 = écart type de la température du groupe contrôle, et
d2 = écart type de la température du groupe échantillon.

Le tableau 2 ci-dessous résume l'indice d'hétérogénéité thermique (h) obtenu dans les régions du tronc inférieur et latérale de la jambe pour le groupe C (athlètes vêtus de tee-shirt et bermuda en fil polyamide 66 contenant 1,5% de Ti02) et D (athlètes vêtus de tee-shirt et bermuda en fil polyamide 66 contenant 1,5% de Ti02 et 0,5% de BaS04).

Tableau 2

| Groupe | Échantillon | h (%) |
|--------|-------------|-------|
| C | Polyamide 66 contenant 1,5% de TiO2 | 20 |
| D | Polyamide 66 TiO2 + BaSO4 | 14 |

Les résultats du tableau 2 ci-dessus montrent une plus grande homogénéité pour le groupe D (indice d'hétérogénéité thermique plus petit), témoignant de l'influence de l'additif favorisant l'homogénéité de la température corporelle.

## Exemple 3

Un échantillon de fil de polyamide 66 contenant 1,5% de Ti02, 0,5% de BaS04 et 0,2% de tourmaline a été préparé selon la description précédente. On a évalué la concentration de lactate (L) dans le sang de deux groupes de 15 athlètes avant et après l'application d'un protocole d'activité physique de test ergométrique en tapis de course selon le protocole de Bruce. La concentration de lactate (en mmol/litre) a été obtenue au moyen de l'analyse strip test (équipement Accutrend lactate de Roche Diagnôstica Brasil).

Le test a été effectué selon les étapes décrites dans l'exemple 1, avec des échantillons de polyester et polyamide 66 contenant 1,5% de Ti02, 0,5% de BaS04 et 0,2% de tourmaline et l'indice de variation de lactate $\Delta L/\Delta L1$ a été calculé:

$$\Delta L = \Delta L2 - \Delta L1$$

où:

$\Delta L1 = L_{fc} - L_{lc}$ (soustraction entre les concentrations de lactate final $L_{fc}$ et initial $L_{lc}$ du groupe contrôle), et
$\Delta L2 = L_{fe} - L_{le}$ (soustraction entre les concentrations de lactate final $L_{fe}$ et initial $L_{le}$ du groupe échantillon).

Le tableau 3 ci-dessous montre l'indice de variation de concentration de lactate OL/OL1 obtenu pour le groupe E (athlètes vêtus de tee-shirt et bermuda en polyester) et F (athlètes vêtus de tee-shirt et bermuda en PA66 contenant 1,5% de Ti02, 0,5% de BaS04 et 0,2% de tourmaline).

Tableau 3

| Groupe | Échantillon | ΔL/L1 (%) |
|--------|-------------|-----------|
| E | Polyester | -31 |
| F | Polyamide 66 + TiO2 + BaSO4 + tourmaline | -36 |

Les résultats montrent une diminution de la concentration de lactate dans le sang, supérieur de 5%dans le groupe F, comparée au groupe E. Le taux de lactate dans le sang est directement associé à la fatigue musculaire.

**Exemple 4**

Un échantillon de fil de polyamide 66 contenant 1,5% de TiO2, 0,5% de BaSO4 et 0,2% de tourmaline a été préparé selon la description précédente et comparé à un échantillon de fil polyamide contenant 1,5% de TiO2. On a évalué la variation thermique des régions du tronc inférieur et latérale de la jambe d'un groupe de 15 athlètes soumis à l'application d'un protocole d'activité physique (test ergométrique en tapis de course à une vitesse constante de 6,5km/h et une inclinaison de 6% durant 30 minutes), pour comparaison. Le test a été effectué au cours de trois jours:

- le jour 1, les athlètes ont été soumis au protocole vêtus de bermuda confectionné en fil polyamide 66 contenant 1,5% de TiO2, 0,5% de BaSO4 et 0,2% de tourmaline;
- le jour 2, les athlètes n'ont pratiqué aucune activité physique;
- le jour 3, les athlètes ont été soumis au protocole vêtus de bermuda confectionné en fil polyamide 66 contenant 1,5% de TiO2.

La température du corps a été mesurée au moyen de la technique de thermographie (équipement Raytec Fluke TiSO Thermal Image) avant et après l'application du protocole, et l'indice d'hétérogénéité thermique a été évalué.
L'indice d'hétérogénéité thermique (h) a été obtenu par le calcul de la variation de l'écart des mesures de température dans la région considérée, avant et après l'application du protocole d'activité physique:

$$h = (d2 - d1) / d1$$

où,

d1 = écart type de la température avant l'application du protocole d'activité physique, et
d2 = écart type de la température après l'application du protocole d'activité physique.

Tableau 4

| Échantillon | h (%) |
|-------------|-------|
| Polyamide 66 contenant 1,5% de TiO2 | 30 |
| Polyamide 66 TiO2 + BaSO4 + tourmaline | 20 |

Les résultats du tableau 4 ci-dessus montrent une plus grande homogénéité pour l'échantillon de polyamide 66 contenant du TiO2, du BaSO4 et de la tourmaline (indice d'hétérogénéité thermique plus petit), témoignant de l'influence de l'additif favorisant l'homogénéité de la température corporelle.
L'augmentation de la circulation sanguine, la diminution du niveau de lactate dans le sang et la meilleure homogénéité thermique sont associées à une diminution de fatigue musculaire. Ainsi, les résultats présentés dans les exemples ci-dessus indiquent une diminution de fatigue musculaire en rapport avec l'usage de l'article produit avec les fils de polyamide additivé par l'emploi de charges minérales émetteurs d'infrarouge distant définis dans la présente invention.

## Exemples 5 et 6

Un échantillon de fil de polyamide 66 contenant 1,5% de Ti02 (exemple 5), et un échantillon de fil de polyamide 66 contenant 1,5% de Ti02, 0,5% de BaS04 et 0,2% de tourmaline (exemple 6) ont été préparés selon la description précédente.

Homogénéité thermique de la peau

On a mesuré la variation thermique de la peau (sur une région comprenant le côté extérieur , l'avant et l'arrière de la cuisse et de la fesse) d'un groupe de 15 volontaires ayant porté pendant 60 jours, 6 heures par jour, un bermuda dont une jambe a été fabriquée à l'aide du fil de l'exemple 5, et l'autre jambe a été fabriquée à l'aide du fil de l'exemple 6. Les 15 volontaires présentent un degré de cellulite I ou II sur l'échelle « NURNBERGER-MULLER SCALE » (Référence : Nurnberger F, Muller G. So-called cellulite: an invented disease. J Dermatol Surg Oncol 1978; 4: 221-229).

La température du corps a été mesurée au moyen de la technique de thermographie (équipement Raytec Fluke TiSO Thermal Image) avant et après les 60 jours.

L'indice d'hétérogénéité thermique (h) a été obtenu par le calcul du rapport entre les écarts type de température sur la région considérée avant et à la suite du port de 60 jours du bermuda :

$$h = X_2/X_1$$

où,

$X_1$ = écart type de la température sur la région (moyenne des écart type pour les 15 volontaires) avant les 60 jours, et
$X_2$ = écart type de la température sur la région (moyenne des écart type pour les 15 volontaires) après les 60 jours

Le tableau 1 ci-dessous résume l'indice d'hétérogénéité thermique (h) obtenu pour le fil polyamide 66 contenant 1,5% de Ti02 et pour le polyamide 66 contenant 1,5% de Ti02, 0,5% de BaS04 et 0,2% de tourmaline.

<div align="center">Tableau 5</div>

| Échantillon | h (%) |
|---|---|
| Polyamide 66 contenant 1,5% de TiO2 | 0,84 |
| Polyamide 66 TiO2 + BaSO4 + tourmaline | 0,67 |

Les résultats du tableau 5 ci-dessus montrent qu'il y a eu une diminution de 33% de l'hétérogénéité thermique suite au port du bermuda pour le fil polyamide 66 contenant 1,5% de Ti02, 0,5% de BaS04 et 0,2% de tourmaline, et une diminution de 16% de l'hétérogénéité thermique suite au port du bermuda pour le fil polyamide 66 contenant 1,5% de Ti02. Ces résultats montrent l'influence de l'additif favorisant l'homogénéité de la température corporelle.

Elasticité de la peau

Outre la variation thermique du corps, on mesure également la variation d'élasticité de la peau du même groupe de volontaires (sur une région comprenant le côté extérieur, l'arrière et l'avant de la cuisse et de la fesse) avant et après les 60 jours.

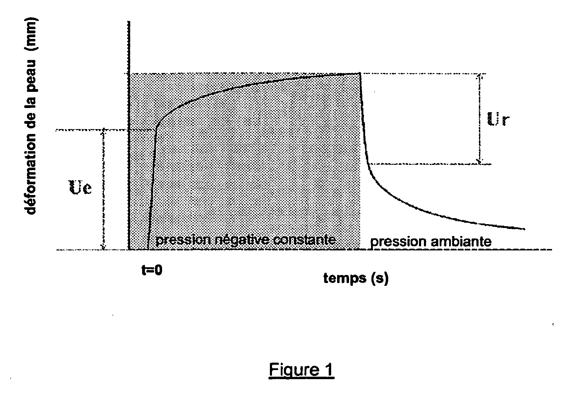

L'élasticité de la peau a été mesurée à l'aide de l'appareil Cutometer ® MPA580 commercialisé par la société CK Electronic GmbH qui utilise le principe de la méthode de succion. Une pression négative est créée dans l'appareil et la peau est tirée dans l'ouverture de la sonde. A l'intérieur de la sonde, on mesure la profondeur de pénétration de la sonde. La capacité de la peau à retourner à sa position initiale lorsque la pression négative n'est plus appliquée (élasticité) est mesurée et exprimée à l'aide d'une courbe (voir figure 1).

L'élasticité de la peau correspond au rapport $R=U_r/U_e$, les valeurs de $U_r$ et $U_e$ correspondant aux valeurs indiquées sur la figure 1.

La variation d'élasticité F correspond à $F=[(R_f-R_i)/R_i]*100$ avec $R_i$ qui correspond à l'élasticité avant les 60 jours, et $R_f$ qui est l'élasticité après les 60 jours.

Le tableau 6 ci-dessous résume la variation d'élasticité F obtenue pour le fil polyamide 66 contenant 1,5% de Ti02 et pour le polyamide 66 contenant 1,5% de Ti02 (exemple 5) et 0,5% de BaS04 et 0,2% de tourmaline (exemple 6).

Tableau 6

| Échantillon | F (%) |
|---|---|
| Polyamide 66 contenant 1,5% de TiO2 | 1 |
| Polyamide 66 TiO2 + BaSO4 + tourmaline | 8 |

Les résultats du tableau 2 ci-dessus montrent une augmentation significative d'élasticité pour le fil polyamide 66 contenant 1,5% de Ti02, 0,5% de BaS04 et 0,2% de tourmaline que pour le fil polyamide 66 contenant 1,5% de Ti02. Ces résultats montrent l'influence de l'additif favorisant l'augmentation de l'élasticité de la peau et donc la diminution de la cellulite.

L'augmentation de l'élasticité de la peau et la meilleure homogénéité thermique sont associées à un meilleur confort et bien-être, et à une diminution de la cellulite. Ainsi, les résultats présentés dans les exemples ci-dessus indiquent un meilleur confort et bien-être en rapport avec l'usage de l'article produit avec les fils de polyamide additivé par l'emploi de charges minérales émetteurs d'infrarouge distant définis dans la présente invention.

Activité antibactérienne

L'activité antibactérienne est mesurée selon la norme JIS L 1902 : 2002 sur les bactéries *Staphylococcus aureus* ATCC 6538P et *Klebsiella Pneumoniae ATCC4352* pour des fils de polyamide 66 contenant 1,5% de Ti02, 0,5% de BaS04 et 0,2% de tourmaline (exemple 6) :

Ladite activité est également comparée à des fils de coton n'ayant pas d'additif.

Les tests sont effectués sur des surfaces tricotées de 0,4g.

Les différences surfaces des échantillons sont mises en contact avec le même nombre de bactéries, pour incubation à 37°C pendant 18 heures. Au temps t=0 et t=18h on procède au décompte du nombre de bactéries.

Après vérification de la bonne croissance des bactéries dans les échantillons témoins, on détermine, pour chaque échantillon, le nombre moyen de bactéries actives immédiatement après l'inoculation sur les différents échantillons et le nombre moyen de bactéries actives après 18 heures d'incubation sur les différents échantillons, en cfu (colony forming unit / unité formant une colonie).

On mesure alors l'activité spécifique bactériostatique S correspondant à la différence entre le logarithme du nombre moyen de bactéries actives après 18 heures d'incubation sur l'échantillon témoin (fils coton sans additif) et le logarithme du nombre moyen de bactéries actives après 18 heures d'incubation sur l'échantillon (fils polyamide 66 de l'exemple 6).

Les résultats sont exprimés dans le tableau 7 :

Tableau 7

| | Activité spécifique bactériostatique S1 *(Staphylococcus aureus)* | Activité spécifique bactériostatique S2 *(Klebsiella Pneumoniae)* |
|---|---|---|
| Fils de l'exemple 6 | 0,76 | 0,48 |

Les résultats du tableau 7 ci-dessus montrent une bonne activité bactériostatique des fils polyamide 66 contenant 1,5% de Ti02, 0,5% de BaS04 et 0,2% de tourmaline. Ces résultats sont très intéressants pour des articles, en particulier des articles textiles, qui sont au contact avec la peau.

## Exemple 7 et 8

Les échantillons des exemples ci-dessous ont été préparés avec un polyamide 66 de viscosité relative (VR) 43, mesurée dans une solution d'acide formique à 90% dans l'eau. L'incorporation du Ti02 et de la tourmaline dans le polyamide 66 est réalisée par introduction de ces charges lors du procédé de polymérisation du polyamide 66, sous la forme d'une suspension aqueuse de Ti02 à 20%, et d'une suspension aqueuse de tourmaline à 39%. L'incorporation du BaSo4 dans le polyamide 66 a été réalisée à travers le mélange des charges minérales sous forme de poudre et du polyamide 66, dans une proportion de 20% en poids de BaS04 pour l'obtention d'un mélange maître. Le mélange a été extrudé, refroidi et granulé. Le « masterbatch » ainsi obtenu a été introduit dans le polyamide 66 au cours de la phase de filage. La composition polymérique fondue a été filée à une température entre 280°C et 300°C (mesurée dans la filière), refroidie à l'air (20°C, humidité relative de 65%) et enroulée à une vitesse de 4200m/min pour obtenir un fil continu multifilamentaire. Le fil multifilamentaire formé de 68 filaments de section circulaire a été postérieurement texturé. Le titre du filament dans le produit fini est de 1,2dtex. Le fil ainsi obtenu a été utilisé dans la production de tricots pour la confection de bermudas, par utilisation d'une tricoteuse circulaire. Les bermudas ainsi obtenus présentent une densité de surface de 305 g/m², et contiennent 12% d'élasthanne. Ces articles ont ensuite été utilisés pour évaluer la performance des compositions.

Un échantillon de fil de polyamide 66 contenant 1,5% de Ti02 (exemple 7), et un échantillon de fil de polyamide 66 contenant 1,5% de Ti02, 0,5% de BaS04 et 0,2% de tourmaline (exemple 8) ont été préparés selon la description précédente.

La micro-circulation sanguine à proximité de la peau a été évaluée par ultrasons à l'aide de la méthode Power Doppler. L'effet Doppler est un changement de fréquence des ultrasons réfléchis par les cellules sanguines en mouvement.

Les tests ont été réalisés sur un groupe de 15 volontaires (ayant un indice de masse corporelle de 23+/-4 kg/m²) ayant porté pendant 60 jours, 6 heures par jour, un bermuda dont une jambe a été fabriquée à l'aide du fil de l'exemple 8 (jambe droite) et l'autre jambe a été fabriquée à l'aide du fil de l'exemple 7 (jambe gauche).

La méthode Power Doppler mesure l'amplitude des signaux Doppler, directement corrélable à la vitesse/intensité du flux sanguin dans la région évaluée.

Les résultats obtenus ont été traités avec un indice de confiance de 95%.

L'augmentation du signal Doppler D% est mesurée, entre le temps t0 (initial) et t60 (après 60 jours).

Elle correspond à la formule suivante :

$$D\% = \frac{\left[\dfrac{\sum_{i=1}^{15}(Dt60_i - Dt0_i)}{n}\right]}{\left[\dfrac{\sum_{i=1}^{15}(Dt0_i)}{n}\right]}$$

dans laquelle D est le signal Doppler, n est égal à 15 (nombre de volontaires)

Tableau 8

| Exemple | Augmentation moyenne du signal Doppler D(%) |
|---|---|
| 7 | 31,1 |
| 8 | 92,0 |

Une augmentation de la micro-circulation sanguine est confirmée pour les deux exemples.

Le tricot préparé à partir de fils de polyamide 66 contenant 1,5% de TiO2, 0,5% de BaSO4 et 0,2% de tourmaline est environ 3 fois meilleur que celui préparé à partir de fils de polyamide 66 contenant 1,5% de Ti02.

Les résultats indiqués ici montrent que l'augmentation de la micro-circulation sanguine est liée à l'utilisation d'un tricot préparé à partir d'un polyamide modifié par des charges minérales absorbant/émettant dans l'infra rouge lointain décrites dans la présente invention.

La composition de l'exemple 8 présente les propriétés d'absorption de radiations infrarouges suivantes :

- nombre de pics dans la plage de fréquence 3,00 +/- 0,30μm : 2
- nombre de pics dans la plage de fréquence 6,20 +/- 0,50μm : 2
- nombre de pics dans la plage de fréquence 8,00 +/- 0,25μm : 1
- nombre de pics dans la plage de fréquence 8,50 +/- 0,25μm : 1
- nombre de pics dans la plage de fréquence 9,00 +/- 0,25μm : 0
- nombre de pics dans la plage de fréquence 9,50 +/- 0,25μm : 1
- nombre de pics dans la plage de fréquence 10,00 +/- 0,25μm : 0
- nombre de pics dans la plage de fréquence 10,50 +/- 0,25μm : 2
- nombre de pics dans la plage de fréquence 11,00 +/- 0,25μm : 0
- nombre de pics dans la plage de fréquence 14,60 +/- 2,10μm : 3

**Revendications**

1. Utilisation d'un article à base d'une composition polymérique comprenant un polymère et au moins deux additifs introduits dans la matrice polymérique choisis parmi les charges minérales qui présentent une capacité d'émission et/ou absorption de radiations infrarouges dans la plage de longueur d'onde située entre $2\mu m$ et $20\mu m$, pour diminuer l'effet « peau d'orange » lié à la cellulite sur la peau de personnes ayant de la cellulite par émission et/ou absorption de radiations infrarouges dans la plage de longueur d'onde située entre $2\ \mu m$ et $20\mu m$ ; les charges minérales étant choisies parmi les oxydes, sulfates, carbonates, phosphates et silicates, et présentant une taille moyenne de particule inférieure ou égale à $2\mu m$ ; la proportion en poids de l'association de charges minérales par rapport au poids total de la composition polymérique étant inférieure à 6 %.

2. Utilisation selon la revendication 1, **caractérisée en ce que** le polymère est choisi parmi les polyesters, les polyoléfines, les polymères cellulosiques tels que l'acétate de cellulose, le propionate de cellulose, le rayon, la viscose, les polymères et copolymères acryliques, et les polyamides en général, comme le polyamide 66 ou le polyamide 6, ou leurs copolymères ou leurs mélanges.

3. Utilisation selon la revendication 2, **caractérisée en ce que** le polymère est à base de polyamide.

4. Utilisation selon la revendication 3, **caractérisée en ce que** le polymère qui compose la matrice de la composition polymérique est un polyamide choisi entre le polyamide 6, le polyamide 66 et les copolymères de polyamide 6/polyamide 66.

5. Utilisation selon l'une des revendications précédentes, **caractérisée en ce que** l'oxyde est choisi parmi le dioxyde de titane, le dioxyde de silicium et l'oxyde de magnésium.

6. Utilisation selon l'une des revendications précédentes, **caractérisée en ce que** le sulfate est choisi parmi le sulfate de baryum, le sulfate de calcium et le sulfate de strontium.

7. Utilisation selon l'une des revendications précédentes, **caractérisée en ce que** le carbonate est choisi parmi le carbonate de calcium ou de sodium.

8. Utilisation selon l'une des revendications précédentes, **caractérisée en ce que** le silicate est choisi parmi l'actinolite, la tourmaline, la serpentine, le kaolin et d'autres aluminosilicates.

9. Utilisation selon l'une des revendications précédentes, **caractérisée en ce que** le phosphate est choisi parmi les phosphates de zirconium, l'apatite ou leurs mélanges.

10. Utilisation selon l'une des revendications précédentes, **caractérisée en ce que** les charges minérales sont choisies parmi le dioxyde de titane, le sulfate de baryum, et une charge du groupe des silicates.

11. Utilisation selon la revendication 10, **caractérisée en ce que** la composition polymérique comprend au moins deux charges minérales choisies parmi le dioxyde de titane, le sulfate de baryum, et la tourmaline.

12. Utilisation selon l'une des revendications précédentes, **caractérisée en ce que** la composition polymérique comprend deux charges minérales.

13. Utilisation selon l'une des revendications précédentes, **caractérisée en ce que** la composition polymérique comprend trois charges minérales.

14. Utilisation selon la revendication 13, **caractérisée en ce que** la proportion en poids des trois charges est comprise entre 80:10:10 et 10:30:60.

15. Utilisation selon la revendication 13 ou 14, **caractérisée en ce que** l'association des trois charges minérales est l'association dioxyde de titane/ sulfate de baryum/ tourmaline

16. Utilisation selon l'une des revendications précédentes, **caractérisée en ce que** la proportion en poids de l'association de charges minérales par rapport au poids total de la composition polymérique est supérieure à 1,0%, de préférence supérieure ou égale à 1,5% et plus préférentiellement encore supérieure ou égale a 2,5%.

**17.** Utilisation selon l'une des revendications précédentes, **caractérisée en ce que** la proportion en poids de l'association de charges minérales par rapport au poids total de la composition polymérique est inférieure à 4,5%.

**18.** Utilisation selon l'une des revendications précédentes, **caractérisée en ce que** l'article se présente sous la forme de fils, fibres, filaments, ou un mélange de ceux-ci, tissus, non tissé ou tricot, ou d'un film ou d'une poudre.

**Patentansprüche**

**1.** Verwendung eines Artikels auf Basis einer Polymerzusammensetzung, umfassend ein Polymer und mindestens zwei in die Polymermatrix eingeführte Additive, die aus mineralischen Füllstoffen mit einem Emissions- und/oder Absorptionsvermögen für Infrarotstrahlung im Wellenlängenbereich zwischen 2 $\mu$m und 20 $\mu$m ausgewählt sind, zur Verringerung des mit Cellulite verbundenen Orangenhaut-Effekts auf der Haut von Personen mit Cellulite durch Emission und/oder Absorption von Infrarotstrahlung im Wellenlängenbereich zwischen 2 $\mu$m und 20 $\mu$m; wobei die mineralischen Füllstoffe aus Oxiden, Sulfaten, Carbonaten, Phosphaten und Silikaten ausgewählt sind und eine mittlere Teilchengröße kleiner gleich 2 $\mu$m aufweisen; wobei der Gewichtsanteil der Kombination von mineralischen Füllstoffen, bezogen auf das Gesamtgewicht der Polymerzusammensetzung, weniger als 6% beträgt.

**2.** Verwendung nach Anspruch 1, **dadurch gekennzeichnet, dass** das Polymer aus Polyestern, Polyolefinen, Cellulosepolymeren wie Celluloseacetat, Cellulosepropionat, Reyon, Viskose, Acrylpolymeren und -copolymeren und Polyamiden im Allgemeinen, wie Polyamid 66 oder Polyamid 6, oder deren Copolymeren oder deren Gemischen ausgewählt ist.

**3.** Verwendung nach Anspruch 2, **dadurch gekennzeichnet, dass** das Polymer auf Polyamid basiert.

**4.** Verwendung nach Anspruch 3, **dadurch gekennzeichnet, dass** es sich bei dem Polymer, das die Matrix der Polymerzusammensetzung ausmacht, um ein Polyamid handelt, das aus Polyamid 6, Polyamid 66 und den Copolymeren von Polyamid 6/Polyamid 66 ausgewählt ist.

**5.** Verwendung nach einem der vorhergehenden Ansprüche, **dadurch gekennzeichnet, dass** das Oxid aus Titandioxid, Siliciumdioxid und Magnesiumoxid ausgewählt ist.

**6.** Verwendung nach einem der vorhergehenden Ansprüche, **dadurch gekennzeichnet, dass** das Sulfat aus Bariumsulfat, Calciumsulfat und Strontiumsulfat ausgewählt ist.

**7.** Verwendung nach einem der vorhergehenden Ansprüche, **dadurch gekennzeichnet, dass** das Carbonat aus Calcium- oder Natriumcarbonat ausgewählt ist.

**8.** Verwendung nach einem der vorhergehenden Ansprüche, **dadurch gekennzeichnet, dass** das Silikat aus Actinolit, Turmalin, Serpentin, Kaolin und anderen Aluminosilikaten ausgewählt ist.

**9.** Verwendung nach einem der vorhergehenden Ansprüche, **dadurch gekennzeichnet, dass** das Phosphat aus Zirconiumphosphaten und Apatit oder deren Mischungen ausgewählt ist.

**10.** Verwendung nach einem der vorhergehenden Ansprüche, **dadurch gekennzeichnet, dass** die mineralischen Füllstoffe aus Titandioxid, Bariumsulfat und einem Füllstoff aus der Gruppe der Silikate ausgewählt ist.

**11.** Verwendung nach Anspruch 10, **dadurch gekennzeichnet, dass** die Polymerzusammensetzung mindestens zwei mineralische Füllstoffe, die aus Titandioxid, Bariumsulfat und Turmalin ausgewählt sind, umfasst.

**12.** Verwendung nach einem der vorhergehenden Ansprüche, **dadurch gekennzeichnet, dass** die Polymerzusammensetzung zwei mineralische Füllstoffe umfasst.

**13.** Verwendung nach einem der vorhergehenden Ansprüche, **dadurch gekennzeichnet, dass** die Polymerzusammensetzung drei mineralische Füllstoffe umfasst.

**14.** Verwendung nach Anspruch 13, **dadurch gekennzeichnet, dass** der Gewichtsanteil der drei Füllstoffe zwischen 80:10:10 und 10:30:60 liegt.

**15.** Verwendung nach Anspruch 13 oder 14, **dadurch gekennzeichnet, dass** es sich bei der Kombination der drei Füllstoffe um die Kombination Titandioxid/Bariumsulfat/Turmalin handelt.

**16.** Verwendung nach einem der vorhergehenden Ansprüche, **dadurch gekennzeichnet, dass** der Gewichtsanteil der Kombination von mineralischen Füllstoffen, bezogen auf das Gesamtgewicht der Polymerzusammensetzung, größer als 1,0%, vorzugsweise größer gleich 1,5% und noch weiter bevorzugt größer gleich 2,5% ist.

**17.** Verwendung nach einem der vorhergehenden Ansprüche, **dadurch gekennzeichnet, dass** der Gewichtsanteil der Kombination von mineralischen Füllstoffen, bezogen auf das Gesamtgewicht der Polymerzusammensetzung, weniger als 4,5% beträgt.

**18.** Verwendung nach einem der vorhergehenden Ansprüche, **dadurch gekennzeichnet, dass** der Artikel in Form von Fäden, Fasern, Filamenten oder einem Gemisch davon, Geweben, Vliesstoffen oder Maschenwaren, oder eines Films oder eines Pulvers vorliegt.

## Claims

**1.** Use of an article based on a polymeric composition comprising a polymer and at least two additives introduced into the polymeric matrix, chosen from inorganic fillers which have a capacity for emission and/or absorption of infrared radiation in the wavelength range of between 2 $\mu$m and 20 $\mu$m, for reducing the "orange peel" effect associated with cellulite on the skin of individuals who have cellulite, by emission and/or absorption of infrared radiation in the wavelength range of between 2 $\mu$m and 20 $\mu$m; the inorganic fillers being chosen from oxides, sulphates, carbonates, phosphates and silicates, and having an average particle size of less than or equal to 2 $\mu$m; the proportion by weight of the combination of inorganic fillers relative to the total weight of the polymeric composition being less than 6%.

**2.** Use according to Claim 1, **characterized in that** the polymer is chosen from polyesters, polyolefins, cellulose-based polymers such as cellulose acetate, cellulose propionate, rayon, viscose, acrylic polymers and copolymers, and polyamides in general, such as polyamide 66 or polyamide 6, or copolymers thereof or blends thereof.

**3.** Use according to Claim 2, **characterized in that** the polymer is based on polyamide.

**4.** Use according to Claim 3, **characterized in that** the polymer of which the matrix of the polymeric composition is composed is a polymer chosen from polyamide 6, polyamide 66 and polyamide 6/ polyamide 66 copolymers.

**5.** Use according to one of the preceding claims, **characterized in that** the oxide is chosen from titanium dioxide, silicon dioxide and magnesium oxide.

**6.** Use according to one of the preceding claims, **characterized in that** the sulphate is chosen from barium sulphate, calcium sulphate and strontium sulphate.

**7.** Use according to one of the preceding claims, **characterized in that** the carbonate is chosen from calcium carbonate or sodium carbonate.

**8.** Use according to one of the preceding claims, **characterized in that** the silicate is chosen from actinolite, tourmaline, serpentine, kaolin and other aluminosilicates.

**9.** Use according to one of the preceding claims, **characterized in that** the phosphate is chosen from zirconium phosphates and apatite, or mixtures thereof.

**10.** Use according to one of the preceding claims, **characterized in that** the inorganic fillers are chosen from titanium dioxide, barium sulphate and a filler of the silicate group.

**11.** Use according to Claim 10, **characterized in that** the polymeric composition comprises at least two inorganic fillers chosen from titanium dioxide, barium sulphate and tourmaline.

**12.** Use according to one of the preceding claims, **characterized in that** the polymeric composition comprises two inorganic fillers.

13. Use according to one of the preceding claims, **characterized in that** the polymeric composition comprises three inorganic fillers.

14. Use according to Claim 13, **characterized in that** the proportion by weight of the three fillers is between 80:10:10 and 10:30:60.

15. Use according to Claim 13 or 14, **characterized in that** the combination of the three inorganic fillers is the titanium dioxide/barium sulphate/ tourmaline combination.

16. Use according to one of the preceding claims, **characterized in that** the proportion by weight of the combination of inorganic fillers relative to the total weight of the polymeric composition is greater than 1.0%, preferably greater than or equal to 1.5% and even more preferentially greater than or equal to 2.5%.

17. Use according to one of the preceding claims, **characterized in that** the proportion by weight of the combination of inorganic fillers relative to the total weight of the polymeric composition is less than 4.5%.

18. Use according to one of the preceding claims, **characterized in that** the article is in the form of yarns, fibres, filaments, or a mixture thereof, fabrics, a nonwoven or a knit, or of a film or of a powder.

Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5053275 A
- US 6316102 B
- US 7201945 B
- EP 1792724 A
- US 4999243 A
- US 5880044 A
- WO 2007055432 A
- EP 1094136 A

**Littérature non-brevet citée dans la description**

- **NIWA, Y. ; LIZAWA O. ; ISHIMOTO K. ; JIANG. X. ; KANOH, T. et al.** Electromagnetic Wave Emitting products and ''Kikoh'' Potentiate Human Leukocyte Functions. *International Journal of Biometeorology,* 1993, 133-138
- **PÉREZ, A. C. N. ; MARTINEZ, A. J. A.** Fibra de Photon Plantino. *Saint Jean de Compostelle,* 1995, 7-71
- Référence : Nurnberger F, Muller G. So-called cellulite: an invented disease. *J Dermatol Surg Oncol,* 1978, vol. 4, 221-229